# EUROPEAN PATENT SPECIFICATION

(11) **EP 1 488 748 B1**
(45) Date of publication and mention of the grant of the patent: **22.04.2009**
(21) Application number: 04014198.8
(22) Date of filing: 17.06.2004
(51) Int. Cl.: A61B 17/22

(54) **Living body lumen washing apparatus**
Waschvorrichtung von Lumen lebendiger Körper
Appareil de lavage d'une lumière d'un corps vivant

(30) Priority: 17.06.2003 JP 2003172621
(43) Date of publication of application: 22.12.2004
(73) Proprietor: TERUMO KABUSHIKI KAISHA, kanagawa, 259-0151 (JP)
(72) Inventor: Maki, Shin Terumo Kabushiki Kaisha, Kanagawa 259-0151 (JP); Ishiyama, Haruo Terumo Kabushiki Kaisha, Kanagawa 259-0151 (JP)
(74) Representative: Casalonga, Axel

(56) References cited:
- WO-A-02/15807
- US-A- 4 950 238
- US-A- 5 947 985
- US-A- 6 129 698
- US-A1- 2001 004 692
- PATENT ABSTRACTS OF JAPAN vol. 0143, no. 42 (C-0743), 24 July 1990 (1990-07-24) & JP 2 124128 A (FUJIKURA LTD), 11 May 1990 (1990-05-11)

## Description

### FIELD OF THE INVENTION

This invention generally relates to a device for removing substance from a lumen. More particularly, the present invention pertains to a living body lumen washing apparatus for removing lipid encapsulated in the lumen wall to clean the inside of the living body lumen such as, for example, the removal of plaque produced in a heart blood vessel (for example in a coronary artery) to wash the blood vessel lumen. The closest prior art is US-A-5 947 985, which defines the preamble of claim 1. In addition, US-A-6 129 698 discloses a living body lumen washing apparatus comprising means for applying liquid pressure to a lumen wall in order to obtain a cleaning effect, the apparatus also comprising a motor coupled by a shaft to a distal impeller for inducing thrombus or other occlusive material into the catheter body.

### BACKGROUND DISCUSSION

Various types of ischemic heart diseases are known, for example a stricture of the heart caused by a stricture of a coronary artery and advanced myocardial infarction. A stricture of a blood vessel is caused by lipid produced in the blood vessel wall and plaque is produced by an increase of the lipid and a succeeding tissue reaction. Arteriosclerosis (atheroma) is then formed by accumulation of the lipid to constrict the blood vessel lumen. Heretofore, it has been considered that ischemic heart diseases originate from a stricture or occlusion of a high degree by formation of atheroma in a coronary artery.

In recent years, a relatively novel doctrine has been reported by research workers. According to this theory, some acute strictures of the heart are caused by rupture of plaque. Although several views have been presented indicating that plaque rupture is caused by inflammation or by a physical stimulus during blood vessel intratreatment, no established theory has been developed.

Plaque which is easy to rupture as generally described above is called vulnerable plaque and accumulates in the blood vessel lumen. This is, therefore, different from incrassate atheroma in that it cannot be discriminated or detected even by an angiography inspection. It is considered, however, that when vulnerable plaque ruptures, the lipid infiltrates into the blood and forms a core to start coagulation of the blood to thereby form a thrombus which occludes the blood vessel lumen to cause acute myocardial infarction to develop.

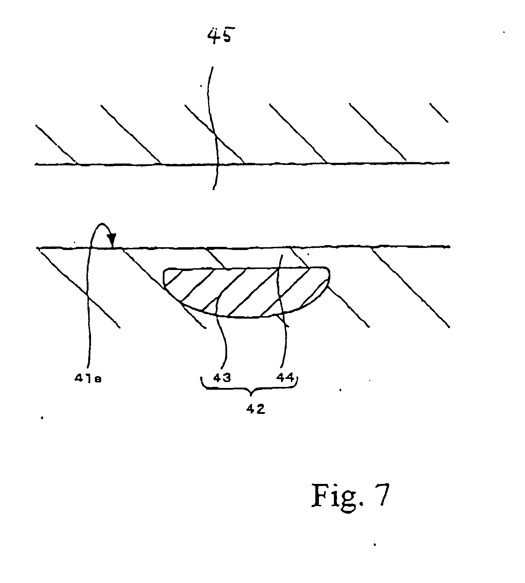

As shown in Fig. 7, vulnerable plaque 42 formed from lipid 43 is included or located in a blood vessel wall 41a, with a thin wall surface (fibrous cap) 44 covering the surface of the lipid 43. As the thin wall surface 44 ruptures, the lipid 43 in the wall infiltrates into the blood vessel 45 and can form a thrombus.

An article entitled "The Coming of Age of Vulnerable Plaque" in the November 2000 issue of START-UP (volume 5, Number 10, pgs 19-23) discusses the possibility of developing acute myocardial infarction from vulnerable plaque and the need for a diagnosis and treatment method.

Removing plaque such as described above in advance, and washing or otherwise cleaning the blood vessel lumen, can help prevent development of acute myocardial infarction arising from a rupture of plaque.

Also, considering living body lumen walls other than blood vessel walls, if lipid encapsulated in the lumen wall can be removed to wash the lumen wall, it may be possible to help inhibit the development of symptoms and/or inhibit expansion of the symptoms.

### SUMMARY

Generally speaking, a living body lumen washing apparatus is provided which makes it possible to remove lipid encapsulated in the lumen wall to clean the living body lumen such as, for example, by removing plaque and washing a blood vessel lumen.

A living body lumen washing apparatus according to the invention is defined in claim 1 and includes a catheter body adapted to be inserted into a living body lumen and a removing device for generating a liquid pressure in the living body lumen to remove lipid encapsulated in the lumen wall.

Liquid pressure is generated in the living body lumen by the removing device provided on the catheter body and the lipid encapsulated in the lumen wall can be removed with the living body lumen being washed.

The removing device may be formed from an agitation device which applies liquid pressure to the lumen wall. The catheter body may also a device for applying vibrational energy to the lumen wall to further facilitate or enhance the cleaning effect. In addition, the catheter body may include a wash introducer for introducing wash into the living body lumen to also facilitate or enhance the cleaning effect.

Different types of wash can be used, including a solution containing a surface-active agent or a solution containing a radiopaque contrast agent. Also, a solution containing liposomes can be used.

The catheter body may be provided with an observation device for observing the state of the lumen wall and/or the dispersion state of removed lipid. It is also possible to provide an arrangement for blocking removed lipid that is of a size sufficient to occlude the living body lumen. This can be in the form of a capturing device for capturing removed lipid of the size sufficient to occlude the lumen. Alternatively, the apparatus may comprise a mechanism for pulverizing the lipid after removed.

The apparatus can be used in connection with a living body lumen in the form of a blood vessel, with the living body lumen washing apparatus being used to wash and remove lipid encapsulated in the blood vessel wall. The liquid pressure generated by the agitation device may be a liquid pressure generated by agitation of the wash introduced into the blood vessel or by agitation of the blood.

### BRIEF DESCRIPTION OF THE DRAWING FIGURES

The foregoing and additional features and characteristics of the present invention will become more apparent from the following detailed description considered with reference to the accompanying drawing figures in which like reference numerals designate like elements.

Fig. 1 is a side view of a living body lumen washing apparatus according to one disclosed embodiment.

Fig. 2 is a perspective view illustrating operation of the living body lumen washing apparatus shown in Fig. 1.

Fig. 3 is a side view of a living body lumen washing apparatus according to another disclosed embodiment.

Fig. 4 is a side view of a living body lumen washing apparatus according to another disclosed embodiment.

Fig. 5 is a side view of a portion of a living body lumen washing apparatus according to a still further disclosed embodiment.

Fig. 6 is perspective view of another portion of the living body lumen washing apparatus.

Fig. 7 is a schematic cross-sectional view of a lumen illustrating a vulnerable plaque.

### DETAILED DESCRIPTION

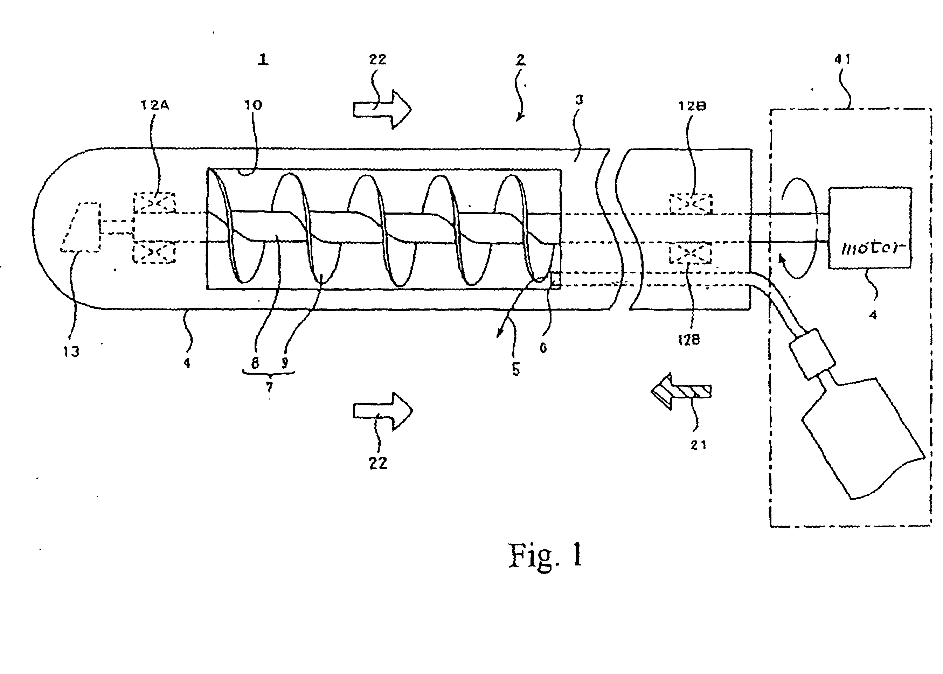

Fig. 1 illustrates one version of a living body lumen washing apparatus according to the disclosed subject matter. The apparatus is applicable to removal of so-called vulnerable plaque (hereinafter referred to simply as plaque) produced or present in a blood vessel wall, and washing of the blood vessel wall.

Generally speaking, the living body lumen washing apparatus 1 includes a catheter 2 (washing catheter) integrally provided with liquid pressure generating means or removing means for generating a liquid pressure in a blood vessel (for example in a coronary artery) to remove lipid encapsulated or located in the blood vessel wall and washing the blood vessel lumen, and a control apparatus 41 for controlling the liquid pressure generating means or removing means of the catheter 2.

More specifically, the catheter 2 includes a catheter body 3 adapted to be inserted into a blood vessel and, by way of example, an agitation device 7 serving as the removing means, with the agitating device being provided in the catheter body 3. The agitation device 7 agitates liquid, for example wash introduced into the blood vessel, to generate a liquid flow which may flow adversely or in opposition to the blood flow direction so that a liquid pressure generated by the adverse flow is applied to the blood vessel wall in order to raise the washing effect. Also, it is possible for the agitation device 7 to agitate the blood by itself, without introduction of a wash or other liquid, so that part of the blood flows adversely to or in opposition to the blood flow direction, thereby applying a liquid pressure generated by the adverse flow to the blood vessel wall. In this disclosed embodiment, a wash introducer 6 is provided for introducing wash 5 into the blood vessel to raise the washing effect.

As shown in Fig. 1, the agitation device 7 can take the form of a screw 9 made of a soft material. This screw 9 may be formed integrally at the distal end of a rotary shaft 8. The screw 9 forms a member which extends outwardly from the shaft 8 to facilitate generation of liquid pressure in the living body lumen. The rotary shaft 8 may be formed from, for example, a closed coil supported for rotation by bearings 12A, 12B. The rotary shaft 8 is also connected to a motor 4 operating as a rotation means to rotate the shaft 8 and thus the screw 9.

The rotary shaft 8 on which the screw 9 is integrally provided to form a single unit is positioned in a central lumen of the catheter body 3. Further, the wash 5 is supplied through a working lumen. In this instance, a wash introducer 6 serves as the working lumen through which the wash is supplied. One or more openings 10 are provided along the portion of the catheter body 3 corresponding to the location of the screw 9 so that the screw 9 and the open end of the working lumen are exposed through the opening(s) 10. By way of example, the catheter body 3 can be provided with two openings 10 positioned in a mutually opposing relationship to each other in a circumferential direction of the catheter body 3. That is, the two openings are located diametrically opposite one another on the catheter body.

The screw 9 is configured such that it introduces the wash 5 into a blood vessel and generates during rotation a liquid flow of the wash 5 directed in the opposite direction to the direction of blood flow. Plaque in the blood vessel wall, particularly the fibrous cap 44 (shown in Fig. 7) of the vessel wall covering lipid 43, is ruptured by the liquid pressure of the wash flow generated by the agitation produced the rotation of the screw 9 so that lipid 43 is removed from the lumen wall.

The catheter body 3 can also be provided with an observation device 13 to permit observation of the state of the blood vessel wall or a dispersion state of lipid removed from the blood vessel wall in the blood vessel, or both. The observation device 13 can be located at the distal end portion of the catheter body 3 and may be in the form of, for example, an endoscope, an ultrasonic transducer for ultrasonic image diagnosis, or the like. The observation device 13 can be connected to a monitor through the working lumen. Also it is possible to provide, based on observation information of the observation device 13, a control section for feeding back the information to the agitation device 7 serving as the removing means or liquid pressure generating means.

The wash 5 is provided for promoting the washing effect. A variety of washes can be used including, for example, a solution containing surface-active agent, a solution containing radiopaque contrast agent, a solution containing liposomes, a solution containing liposomes in which such agent is encapsulated, a saline, and a solution of two or more of the surface-active agent, radiopaque contrast agent, liposomes, micelles, liposomes encapsulating agent and saline. As preferable examples, a liquid wherein, for example, surface-active agent is contained as an agent for promoting solubilization of the thin wall section in saline or solution of contrast agent or liquid wherein, for example, a substance which forms micelles or liposomes is contained as an agent for dispersing removed lipid can be used. The surface-active agent, micelles and liposomes are agents having the oleophilic property. Representative examples of surface-active agents which are used for solubilization of the thin wall section and which can be applied here are listed in Table 1 below.

**[Table 1]**

| Representative surface-active agents used for solubilization of thin wall section | |
|---|---|
| Chemical name | Proprietary Name |
| Sorbitan monostearate | Emasol 310 |
| Sorbitan monolaurate | Span 20 |
| Polyoxyethylene stearyl ether | Brij 76 |
| Polyoxyethylene oleyl ether | Brij 96 |
| Polyoxyethyline nonyl phenyl ether | Triton N-101 |
| Sodium oleate | |
| Oleic acid | |

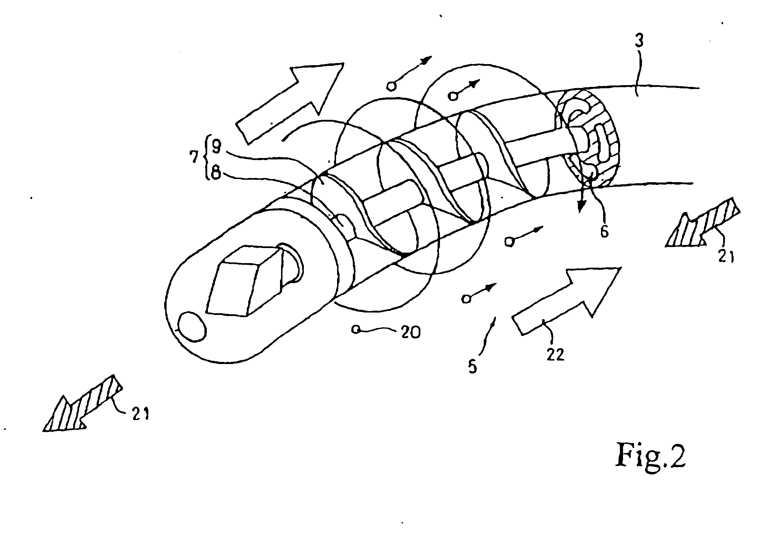

Set forth below is a description of one example of a use in which the living body lumen washing apparatus 1 according to the embodiment described above can be applied. In this example described with reference to Fig. 2, the apparatus 1 is used to remove plaque produced or present in a coronary artery and to wash the coronary artery.

The catheter body 3 is inserted into a coronary artery until the screw 9 (agitation device 7) on the distal end side of the catheter body 3 is positioned at a position generally corresponding to the location of plaque to be removed. The control apparatus 41 is activated to introduce solution containing, for example, liposomes (vesicle membranes similar to cells of a diameter from 10 nm to 10 µm made of phospholipid which forms biomembranes) 20 into the coronary artery through the working lumen forming the wash introducer 6 and to rotate the screw 9 through operation of the motor. By virtue of rotation of the screw 9, the wash 5 (i.e., the solution containing liposomes) is agitated so that it flows along a flow direction (hereinafter referred to as agitation flow) 22 by the rotation of the screw 9, with this flow direction or agitation flow being opposite to or against the direction of blood flow 21. Although the screw 9 does not contact the blood vessel wall, the fibrous cap forming plaque in the blood vessel wall is ruptured and lipid is scraped out or removed by the liquid pressure associated with the agitation flow 22 of the liposomes 20 and the wash 5. Further, because the apparatus 1 is preferably constructed and operated so that the agitation flow 22 is counter or opposite to the blood flow 21, the agitation effect of the wash is further raised or increased, thus facilitating or promoting the lipid scraping out/removing effect. The scraped out or removed lipid is taken into so-called microliposomes and granulated. After the control apparatus 41 is deactivated and the catheter body 3 is removed from the coronary artery, lipid granulated by the microliposomes 20 flows together with the blood and is processed and naturally metabolized by the hepar. In this manner, plaque produced in or present in the blood vessel wall of the coronary artery can be removed to wash the coronary artery.

The state of the blood vessel wall before the washing process can be observed using the observation device 13 (e.g., an endoscope or an ultrasonic transducer) provided at the distal end portion of the catheter body 3 to confirm the location of plaque, with the washing process being performed based on the observation information. Alternatively, or in addition, the state of the blood vessel wall during washing and the dispersion state of lipid scraped out or released into the blood vessel can be observed, with the washing process being performed based on the observation information. The washing effect can be further raised by the washing process based on the observation information.

In the above-described embodiment, the agitation device 7 in the form of the screw 9 made of a relatively soft material which generally does not have a had influence on the living body is used as the removing means for performing removal of plaque and washing of the blood vessel wall. However, other alternatives may be employed.

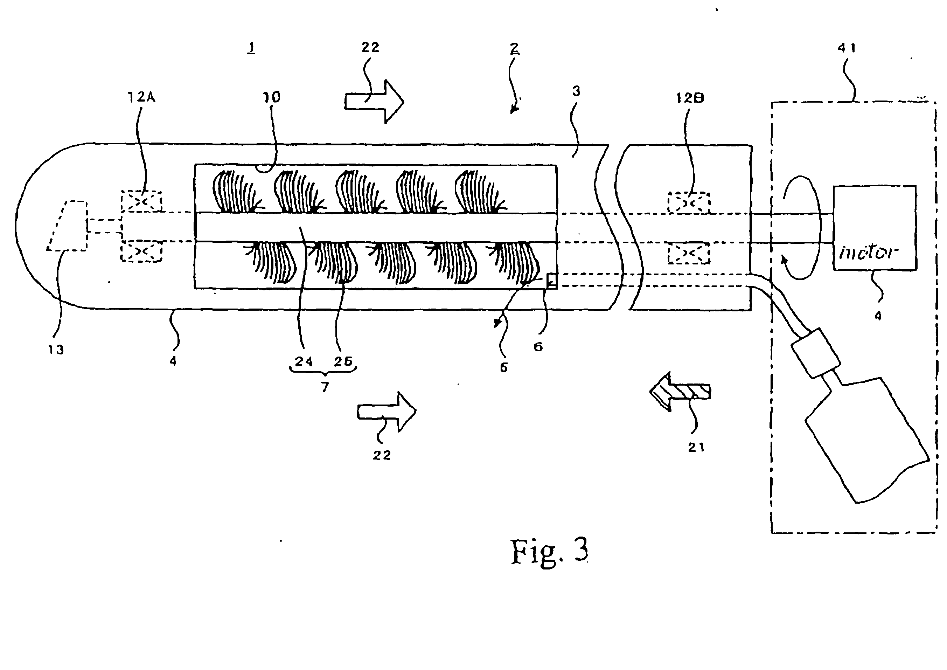

Fig. 3 shows another embodiment of the agitation device serving as the removing means. This embodiment employs an agitation device 7 in which bio-absorbable soft brush-like members or bristles 25 are attached to and disposed around a rotary shaft 24. The bristles 25, which preferably do not contact the lumen wall, form members that extend outwardly from the shaft 24 to facilitate generation of liquid pressure in the living body lumen.

Using this agitation device 7, the brush-like members 25 are rotated through operation of a motor 4 to agitate wash 5 injected into a blood vessel by way of the wash introducer 6 so that the wash 5 may flow adverse to or in opposition to the blood flow direction 21 to remove plaque. Since the brush-like members 25 are formed from bio-absorbable material, they can be absorbed by the living body in the event they fall off, thus not causing harm to the living body.

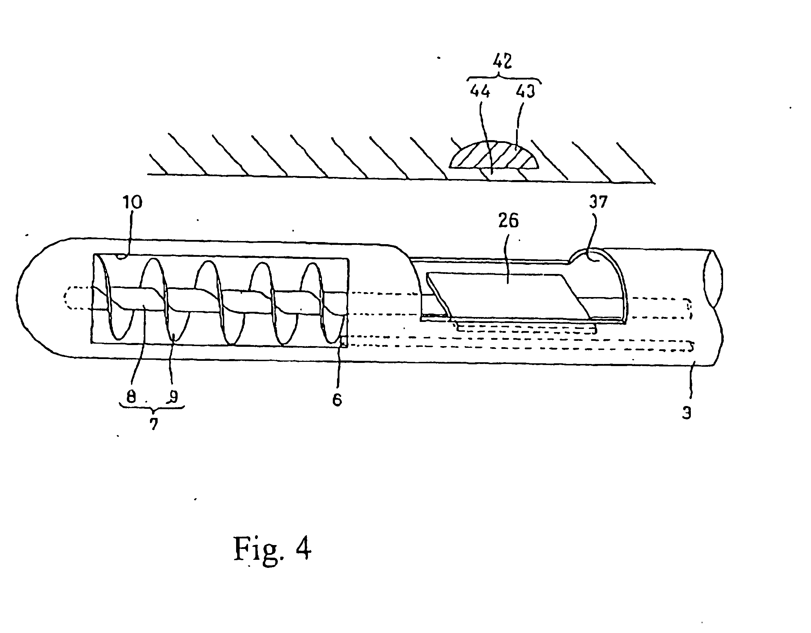

Fig. 4 illustrates another embodiment of the removing means. In this version, vibrational energy application means 26 for further raising the washing effect is provided on the agitation device 7. The screw 9 or the brush-like members 25 described previously can also be applied as the agitation device 7, or jet injection means (described below) can be applied. In the version illustrated in Fig. 4, the agitation device is depicted in the form of the screw 9.

The vibrational energy application means 26 can be in the form of a vibration member 26 formed from, for example, a piezoelectric vibrator, an ultrasonic vibrator or the like. The vibration member 26 is disposed on the front side of the screw 9 (i.e., proximally of the screw) within the catheter body 3. In addition, an opening or widow 37 is provided in the portion of the catheter body 3 corresponding to the location of the vibration member 26.

In this disclosed embodiment, the vibration member 26 is brought to a position corresponding to the location of plaque 42 to be removed, and the blood and the injected wash 5 are vibrated by vibrations generated by the vibration member 26. Vibrational pressure generated by the vibrations is applied to the fibrous cap 44 of plaque 42 to be removed. The fibrous cap 44 is placed into a more readily rupturable state by the vibrational pressure and the liquid pressure generated by the agitation of the screw 9 so that lipid 43 can be scraped out or removed to thus remove plaque 42.

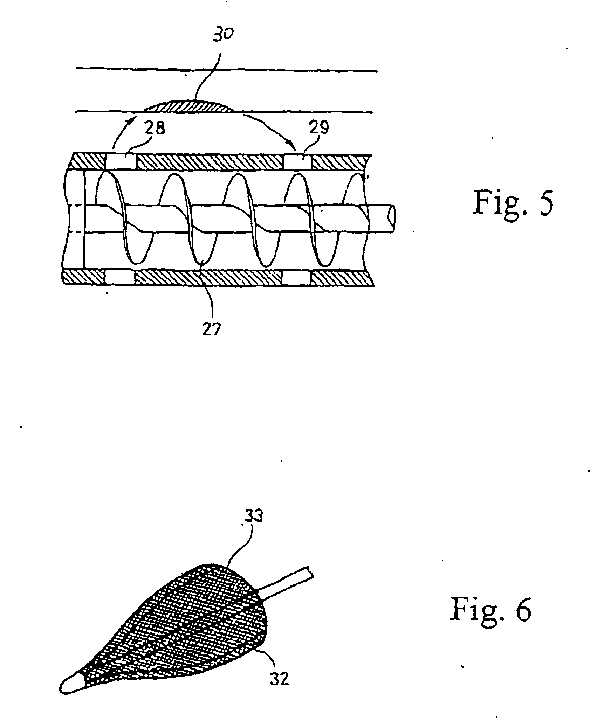

Fig. 5 shows a further embodiment of the removing means. The removing means in this embodiment includes an agitation device as well as a mechanism for avoiding or blocking the flow of removed lipid 43 in a size which might occlude a blood vessel such as, for example, a size sufficient to occlude a peripheral blood vessel. In this embodiment, a device having both an agitation function and a pulverization function is disposed in the catheter body 3. That is, the catheter body 3 is provided with a mixing means 27 in the form of a screw which can rotate to pulverize lipid which is the substance to be removed. In addition, the side wall of the catheter body 3 is provided with blast nozzles 28 for jetting wash 5 toward the blood vessel wall side therethrough while the wash 5 is agitated by the mixing means 27 and suction ports 29 for sucking removed lipid 43 into the catheter body 3. The wash 5 can be blasted or directed from the blast nozzles 28 by rotational driving of the mixing means 27. The wash 5 is placed into an agitated state within the blood vessel and plaque 30 is removed by the agitation and the blasting pressure of the wash 5. Removed lipid 43 is sucked into the catheter body 3 by a negative pressure generated by the rotation of the mixing means 27 and is pulverized minutely within the catheter body 3 by the mixing means 27. Pulverized lipid 43 flows together with the blood and is naturally metabolized after the washing process comes to an end.

Fig. 6 shows a part of another embodiment of the apparatus. This aspect involves means for blocking or inhibiting the flow of removed lipid that is generally of a size sufficient to occlude a lumen, for example lipid of removed plaque that is generally of a size sufficient to occlude a peripheral blood vessel. This means can be formed by a net-like member or filter 32 provided at the distal end of the catheter body 3. The net-like member 32 is formed such that it is closed at the distal end of the catheter body 3 and gradually expands rearwardly from the distal end such that an opening 33 is formed. It is to be understood that the various versions of the removal means described can be used for the agitation device together with the filter.

After plaque is removed by the agitation device as described in the various embodiments discussed above, when a comparatively large lipid mass flows together with the blood flow, the lipid mass can be introduced into the net-like member or filter 32 through the opening 33 and caught by the net-like member 32, thus blocking the lipid mass from flowing to a peripheral blood vessel.

Also, although not specifically shown together, it is possible to include as the removing means a jet injection port and a suction port in the catheter body 3 so that wash is jet-injected from the jet injection port 28, plaque is removed by the jet pressure, and removed plaque is sucked into the catheter body 3 from the suction port(s) 29.

Additionally, though not specifically shown, it is possible to use a soft sponge-like member attached to the catheter body 3 to remove plaque in a generally scraping manner to perform a washing process for the blood vessel wall.

In the embodiments described above, the embodiments of the living body lumen washing apparatus are applied to remove plaque in a coronary artery and wash the blood vessel wall. However, the apparatus can be applied also to the removal of lipid encapsulated in the lumen wall in any other living body lumen than the blood vessels and washing of the inside of the lumen.

By inserting the catheter body 3 into a living body lumen and generating a liquid pressure by the removing means provided in the catheter body 3, lipid encapsulated in the lumen wall can be removed to wash the inside of the lumen. In other words, a new treatment method can be employed by which the development and prevention of expansion of the symptom can be realized by the living body lumen washing apparatus

Using the removing means in the form of the agitating device, liquid pressure sufficient to rupture or more easily rupture the thin wall portion covering the lipid can be applied to the lumen wall without substantially damaging the lumen wall.

Using the vibrational energy application means for applying vibrational energy to the lumen wall together with the removing means or agitation device, the washing effect is further raised or facilitated by the vibrational pressure associated with the vibrational energy, and the treatment involving removal of lipid and washing can be performed without substantially damaging the lumen wall.

When the wash introducer for introducing wash into the living body lumen is provided in addition to the removing means or agitation device, the washing effect is further raised or enhanced by the wash, and the treatment involving removal of lipid and washing can be performed without substantially damaging the lumen wall.

The washing effect can also be increased by using the solution containing a surface-active agent therein, a solution containing a radiopaque contrast agent therein or a solution containing liposomes therein.

Providing the observation means on the catheter body makes it possible to observe or assess the state of the lumen wall and/or the state of dispersion of the removed lipid. Thus, a portion of the lumen wall to be treated can be observed, or while the portion is observed, a lipid removing and washing process can be performed with a higher degree of certainty and the treatment can be performed better.

Using the device for blocking a piece of removed lipid that is of a size sufficient to occlude or inhibit flow through the living body lumen, a piece of lipid which has been removed but is not in a pulverized state can be blocked from moving to some other portion and occluding the lumen. Also, using the capturing means, it is possible to capture a piece(s) of lipid which has been removed but is not in a pulverized state and is generally of a size that might to occlude the lumen. Accordingly, the piece of lipid can be blocked from moving to some other portion and occluding the lumen.

Where apparatus is provided with the pulverizing means for pulverizing removed lipid, lipid of a size generally sufficient to occlude a lumen is blocked from moving to some other portion and occluding the lumen.

Finally, using the living body lumen washing apparatus to remove vulnerable plaque produced or present in the blood vessel wall and perform a washing process for the blood vessel lumen, it is possible to reduce the possibility of an acute stricture of the heart.

The principles, preferred embodiments and modes of operation of the present invention have been described in the foregoing specification. However, the invention which is intended to be protected is not to be construed as limited to the particular embodiments described. Further, the embodiments described herein are to be regarded as illustrative rather than restrictive. Variations and changes may be made by others, and equivalents employed, without departing from the scope of the invention as defined by the appended claims.

## Claims

1. A living body lumen washing apparatus comprising a catheter body (3) positionable in a living body lumen which possesses a lumen wall, the catheter body comprising a lumen, a wash introducer (6) through which wash is introduced into the lumen and at least one opening (10) provided in the catheter body communicating the lumen to outside the catheter body, **characterized in that** the catheter body (3) comprises removing means (8, 9; 24, 25) for generating a wash pressure to remove lipid encapsulated in the lumen wall, said means comprising a rotatable shaft (8; 24) positioned in the lumen and extending along at least a portion of the catheter body;
a member (9; 25) provided on the shaft and extending outwardly from the shaft, the member rotating together with the shaft to generate the wash pressure which passes out through the opening; and
a motor (4) connected to the shaft to rotate the shaft and the member to generate wash pressure sufficient to remove lipid encapsulated in the lumen wall.

2. The living body lumen washing apparatus according to claim 1, wherein the removing means is an agitation device which applies the wash pressure to the lumen wall.

3. The living body lumen washing apparatus according to claim 1 or 2, further comprising means for pulverizing the lipid after removal from the lumen wall.

4. The living body lumen washing apparatus according to any of claims 1 to 3, wherein the catheter body (3) is also provided with vibrational energy application means (26) for applying vibrational energy to the lumen wall.

5. The living body lumen washing apparatus according to any of claims 1 to 4, wherein the member comprises a screw (9) extending helically around the shaft.

6. The living body lumen washing apparatus according to claim 5, wherein the screw (9) is made of a soft material.

7. The living body lumen washing apparatus according to any of claims 1 to 4, wherein the member comprises a plurality of bristles (25) provided on the shaft.

8. The living body lumen washing apparatus according to any of claims 1 to 7, wherein the catheter body (3) is also provided with observation means (13) for observing at least one of a state of the lumen wall and a dispersion state of removed lipid.

9. The living body lumen washing apparatus according to any of claims 1 to 8, wherein the at least one opening in the catheter body comprises a nozzle (28) for directing the wash toward the lumen wall during rotation of the shaft and the member, and at least one section portion (29) for drawing removed lipid into the catheter body during rotation of the shaft and the member.

10. The living body lumen washing apparatus according to any of claims 1 to 9, wherein the living body lumen is a blood vessel in which blood flows in a blood flow direction, and the wash pressure produces an agitation flow opposite the blood flow direction.

11. The living body lumen washing apparatus according to any of claims 1 to 10, wherein the catheter body is also provided with a filter (32) located at a distal end of the catheter body to capture removed lipid.

12. The living body lumen washing apparatus according to claim 1, wherein the living body lumen is a blood vessel possessing a blood vessel wall, and the living body lumen washing apparatus is adapted to remove lipid encapsulated in the blood vessel wall.

## Patentansprüche

1. Spülvorrichtung für ein Lumen eines lebenden Körpers, umfassend einen Kathetertubus (3), welcher in einem Lumen eines lebenden Körpers positionierbar ist, welches eine Lumenwand besitzt, wobei der Kathetertubus ein Lumen, ein Spülmittel-Einleitungsstück (6), wodurch ein Spülmittel in das Lumen eingeleitet wird, und mindestens eine Öffnung (10), welche in dem Kathetertubus vorgesehen ist, welche das Lumen mit der Außenseite des Kathetertubus verbindet, umfaßt, **dadurch gekennzeichnet, daß** der Kathetertubus (3) eine Entfernungseinrichtung (8, 9, 24, 25) zum Erzeugen eines Spüldrucks zum Entfernen von Lipid, welches in der Lumenwand verkapselt ist, umfaßt, wobei die Einrichtung eine drehbare Welle (8; 24) umfaßt, welche in dem Lumen angeordnet ist und sich über mindestens einen Abschnitt des Kathetertubus erstreckt;
ein Element (9; 25), welches an der Welle vorgesehen ist und sich von der Welle ausgehend nach außen erstreckt, wobei sich das Element gemeinsam mit der Welle dreht, um den Spüldruck zu erzeugen, welcher durch die Öffnung nach außen gelangt; und
einen Motor (4), welcher mit der Welle verbunden ist, um die Welle und das Element zu drehen, um einen Spüldruck zu erzeugen, welcher ausreichend ist, um Lipid zu entfernen, welches in der Lumenwand verkapselt ist.

2. Spülvorrichtung für ein Lumen eines lebenden Körpers nach Anspruch 1, wobei die Entfernungseinrichtung eine Rührvorrichtung ist, welche den Spüldruck auf die Lumenwand überträgt.

3. Spülvorrichtung für ein Lumen eines lebenden Körpers nach Anspruch 1 oder 2, ferner umfassend eine Einrichtung zum Pulverisieren des Lipids nach der Entfernung von der Lumenwand.

4. Spülvorrichtung für ein Lumen eines lebenden Körpers nach einem der Ansprüche 1 bis 3, wobei der Kathetertubus (3) ferner mit einer Schwingungsenergie-Anwendungseinrichtung (26) zum Übertragen von Schwingungsenergie auf die Lumenwand versehen ist.

5. Spülvorrichtung für ein Lumen eines lebenden Körpers nach einem der Ansprüche 1 bis 4, wobei das Element eine Schnecke (9) umfaßt, welche helixförmig um die Welle verläuft.

6. Spülvorrichtung für ein Lumen eines lebenden Körpers nach Anspruch 5, wobei die Schnecke (9) aus einem weichen Material hergestellt ist.

7. Spülvorrichtung für ein Lumen eines lebenden Körpers nach einem der Ansprüche 1 bis 4, wobei das Element eine Vielzahl von Borsten (25) umfaßt, welche an der Welle vorgesehen sind.

8. Spülvorrichtung für ein Lumen eines lebenden Körpers nach einem der Ansprüche 1 bis 7, wobei der Kathetertubus (3) ferner mit einer Beobachtungseinrichtung (13) zum Beobachten mindestens eines Zustands der Lumenwand und eines Dispersionszustands des entfernten Lipids versehen ist.

9. Spülvorrichtung für ein Lumen eines lebenden Körpers nach einem der Ansprüche 1 bis 8, wobei die mindestens eine Öffnung in dem Kathetertubus eine Düse (28) zum Lenken des Spülmittels auf die Lumenwand bei einer Drehung der Welle und des Elements und mindestens einen Absaugabschnitt (29) zum Saugen von entferntem Lipid in den Kathetertubus bei einer Drehung der Welle und des Elements umfaßt.

10. Spülvorrichtung für ein Lumen eines lebenden Körpers nach einem der Ansprüche 1 bis 9, wobei das Lumen des lebenden Körpers ein Blutgefäß ist, worin Blut in einer Blutflußrichtung fließt und der Spüldruck eine Rührströmung erzeugt, welche entgegengesetzt zu der Blutflußrichtung verläuft.

11. Spülvorrichtung für ein Lumen eines lebenden Körpers nach einem der Ansprüche 1 bis 10, wobei der Kathetertubus ferner mit einem Filter (32) versehen ist, welcher an einem distalen Ende des Kathetertubus angeordnet ist, um entferntes Lipid aufzufangen.

12. Spülvorrichtung für ein Lumen eines lebenden Körpers nach Anspruch 1, wobei das Lumen des lebenden Körpers ein Blutgefäß ist, welches ein Blutgefäßwand besitzt und die Spülvorrichtung für ein Lumen eines lebenden Körpers geeignet angelegt ist, um Lipid zu entfernen, welches in der Blutgefäßwand verkapselt ist.

## Revendications

1. Appareil de lavage de lumière de corps vivant comprenant un corps de cathéter (3) positionnable dans une lumière d'un corps vivant qui comporte une paroi de lumière, le corps de cathéter comprenant une lumière, un dispositif d'introduction de solution de lavage (6) par lequel on introduit une solution de lavage dans la lumière et au moins une ouverture (10) prévue dans le corps de cathéter, faisant communiquer la lumière avec l'extérieur du corps de cathéter, **caractérisé en ce que** le corps de cathéter (3) comprend un moyen d'élimination (8, 9 ; 24, 25) pour générer une pression de lavage afin de supprimer les lipides incrustés dans la paroi de la lumière, ledit moyen comprenant un axe rotatif (8 ; 24) positionné dans la lumière et s'étendant le long d'au moins une partie du corps de cathéter ;
un élément (9 ; 25) placé sur l'axe et s'étendant vers l'extérieur depuis l'axe, l'élément tournant avec l'axe pour générer la pression de solution de lavage qui sort par l'ouverture ; et
un moteur (4) connecté à l'axe pour faire tourner l'axe et l'élément pour générer une pression de lavage suffisante pour supprimer les lipides incrustés dans la paroi de la lumière.

2. Appareil de lavage de lumière de corps vivant selon la revendication 1, dans lequel le moyen d'élimination est un dispositif d'agitation qui applique la pression de lavage à la paroi de la lumière.

3. Appareil de lavage de lumière de corps vivant selon la revendication 1 ou 2, comprenant en outre un moyen pour pulvériser les lipides après les avoir retirés de la paroi de la lumière.

4. Appareil de lavage de lumière de corps vivant selon l'une quelconque des revendications 1 à 3, dans lequel le corps de cathéter (3) est aussi pourvu d'un moyen d'application d'énergie vibratoire (26) pour appliquer une énergie vibratoire à la paroi de la lumière.

5. Appareil de lavage de lumière de corps vivant selon l'une quelconque des revendications 1 à 4, dans lequel l'élément comprend une vis (9) s'étendant en hélice autour de l'axe.

6. Appareil de lavage de lumière de corps vivant selon la revendication 5, dans lequel la vis (9) est faite d'un matériau mou.

7. Appareil de lavage de lumière de corps vivant selon l'une quelconque des revendications 1 à 4, dans lequel l'élément comprend une pluralité de poils (25) placés sur l'axe.

8. Appareil de lavage de lumière de corps vivant selon l'une quelconque des revendications 1 à 7, dans lequel le corps de cathéter (3) est aussi pourvu d'un moyen d'observation (13) pour observer au moins un état parmi un état de la paroi de la lumière et un état de dispersion des lipides retirés.

9. Appareil de lavage de lumière de corps vivant selon l'une quelconque des revendications 1 à 8, dans lequel chacune desdites ouvertures prévues dans le corps de cathéter comprend une buse (28) pour diriger la solution de lavage vers la paroi de la lumière pendant la rotation de l'axe et de l'élément, et au moins une partie d'aspiration (29) pour amener les lipides retirés dans le corps de cathéter pendant la rotation de l'axe et de l'élément.

10. Appareil de lavage de lumière de corps vivant selon l'une quelconque des revendications 1 à 9, dans lequel la lumière du corps vivant est un vaisseau sanguin dans lequel du sang circule dans une direction d'écoulement sanguin, et la pression de lavage produit un écoulement d'agitation contraire à la direction de l'écoulement sanguin.

11. Appareil de lavage de lumière de corps vivant selon l'une quelconque des revendications 1 à 10, dans lequel le corps de cathéter est aussi pourvu d'un filtre (32) situé à une extrémité distale du corps de cathéter pour capturer les lipides retirés.

12. Appareil de lavage de lumière de corps vivant selon la revendication 1, dans lequel la lumière du corps vivant est un vaisseau sanguin comportant une paroi de vaisseau sanguin, et l'appareil de lavage de lumière de corps vivant est adapté pour supprimer les lipides incrustés dans la paroi du vaisseau sanguin.
